# EUROPEAN PATENT APPLICATION

(11) **EP 1 471 354 A2**
(43) Date of publication of application: **27.10.2004**
(21) Application number: 04252359.7
(22) Date of filing: 22.04.2004
(51) Int. Cl.: G01N 33/52

(54) **Ambidextrous capillary-fillable test strip**

(30) Priority: 23.04.2003 US 465095 P
(71) Applicant: LIFESCAN, INC., Milpitas, California 95035 (US)
(72) Inventor: Matzinger, David P., Menlo Park CA 94025 (US); Ouyang, Tianmei, Fremont CA 94539 (US); Yu, Yeung S., Pleasanton CA 94566 (US)
(74) Representative: Mercer, Christopher Paul

(57) **Abstract**

A test strip for detecting an analyte (e.g., blood glucose) in a liquid sample (e.g., whole blood) includes an analytical portion (e.g., a reagent pad), a first edge with a first edge indentation and a second edge with a second edge indentation. The test strip also includes a first capillary channel adapted for providing fluid communication between the first edge indentation and the analytical portion, as well as a second capillary channel adapted for providing fluid communication between the second edge indentation and the analytical portion. Furthermore, the first and second edges are in an opposing relationship (e.g., on left-hand and right-hand sides of the test strip). Also, a test strip and meter combination for detecting an analyte in a liquid sample. The combination includes a test strip with an analytical portion and first and second edges. The first and second edges of the test strip each include an edge indentation with a sample application region. The test strip also includes a first capillary channel adapted for providing fluid communication between the sample application region of the first edge indentation and the analytical portion, as well as a second capillary channel adapted for providing fluid communication between the sample application region of the second edge indentation and the analytical portion. The meter includes a test strip holder configured to receive the test strip in the absence of contact between the sample application regions of the first and second edge indentations and the test strip holder.

## Description

### BACKGROUND OF THE INVENTION

### 1. Field of the Invention

The present invention relates, in general, to analytical test devices and, in particular, to capillary-filled test strips.

### 2. Description of the Related Art

Various test strips have been developed for detecting the presence and/or concentration of certain analytes in a liquid sample and/or chemical properties (e.g., pH or alkalinity) of a liquid sample. Such test strips can be used for the detection of, for example, glucose, cholesterol, proteins, ketones, phenylalanine or enzymes in a blood, urine or saliva sample. These test strips typically include an analytical portion (e.g., a reagent pad or an electrochemical cell) that facilitates the detection of the analyte concentration or chemical property. Such test strips are described in, for example, U.S. Patent Nos. 4,900,666, 5,902,731 and 5,304,468, each of which is hereby fully incorporated by reference.

Typical monitoring systems for the detection of an analyte or chemical property of a liquid sample include a device, such as a portable hand-held meter, and a test strip to which the liquid sample (e.g., a whole blood sample) is applied. The device and test strip are used in combination to detect an analyte (e.g., determine blood glucose concentration) or other characteristic (e.g., prothrombin time) of the liquid sample. The device typically measures a property or properties of the test strip (e.g., an optical reflectance, optical transmittance, optical absorption or an electrochemical property) and then employs an algorithm(s) to calculate the characteristic based on the measured property or properties.

For the detection of blood glucose in a whole blood sample, a blood sample is most commonly expressed from a user's fingertip and applied to a test strip. The nerve density in the fingertips, however, can cause significant pain in many users, and thus a fingertip may not be the most desirable testing site for those users. Sampling on alternative target sites, such as earlobes, palms, forearms and abdomen, is sometimes practiced since these alternative target sites may be less sensitive. However, regardless of the target site, it is desirable that the test strip requires a relatively small sample volume in order to increase the likelihood of a successful detection of blood glucose. Furthermore, it is desirable that a test strip be easily manipulated by both left-handed and right-handed users.

During application of a liquid sample to a test strip, a portion of the test strip's surface may inadvertently become contaminated with the liquid sample. This contaminated surface can subsequently contaminate a device (e.g., a hand-held test meter) used in tandem with the test strip.

Still needed in the field, therefore, is a test strip that requires a relatively small sample volume, can be easily handled by left and right-handed users and that decreases the likelihood of device contamination.

### SUMMARY OF THE INVENTION

Test strips for the detection of an analyte in a liquid sample according to exemplary embodiments of the present invention require a relatively small sample volume, can be easily handled by left-handed users as well as right-handed users, and decrease the likelihood of device contamination.

An exemplary embodiment of a test strip for detecting an analyte (e.g., blood glucose) in a liquid sample (e.g., a whole blood) according to the present invention includes an analytical portion (e.g., a reagent pad), a first edge with a first edge indentation and a second edge with a second edge indentation. The test strip also includes a first capillary channel adapted for providing fluid communication between the first edge indentation and the analytical portion and a second capillary channel adapted for providing fluid communication between the second edge indentation and the analytical portion. Furthermore, the first and second edges are in an opposing relationship (e.g., on left-hand and right-hand sides of the test strip).

Since test strips according to exemplary embodiments of the present invention include first and second capillary channels that are in fluid communication with opposing edges of the test strip, the test strips can be easily manipulated by both left-handed and right-handed users. In addition, given that a liquid sample is communicated (transported) to the analytical portion by a capillary channel, test strips according to the present invention can employ liquid samples of relatively small volume. Furthermore, as explained in detail below, the presence of first and second edge indentations reduces the likelihood that an associated device (e.g., a meter) will be contaminated by a liquid sample that has been applied to the test strip.

Also provided by the present invention is a test strip and meter combination for detecting an analyte (e.g., determining an analyte concentration) in a liquid sample.
The combination includes a test strip with an analytical portion (e.g., a colorimetric reagent pad), a first edge and a second edge. Each of the first and second edges includes an edge indentation with a sample application region.

The combination also includes a first capillary channel adapted for providing fluid communication between the sample application region of the first edge indentation and the analytical portion, as well as a second capillary channel adapted for providing fluid communication between the sample application region of the second edge indentation and the analytical portion. The meter includes a test strip holder that is configured to receive the test strip in the absence of contact between either the sample application region of the first edge indentation and/or the sample application region of the second edge indentation and the test strip holder. The absence of contact prevents liquid sample contamination of the test strip holder and meter.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings, in which like numbers represent similar elements, of which:

FIG. 1 is a perspective view of a test strip according to an exemplary embodiment of the present invention;

FIG. 2 is an exploded view of the test strip of FIG. 1;

FIG. 3 is a simplified top view of a portion of the test strip of FIGS. 1 and 2, with dashed lines representing elements that are hidden from view;

FIG. 4 is a simplified cross-sectional view of the test strip of FIGS. 1-3 taken along line 4A-4A of FIG. 3;

FIG. 5 is a simplified cross-sectional view of the test strip of FIGS. 1-3 taken along line 5B-5B of FIG. 3;

FIG. 6 is a simplified cross-sectional view of the test strip of FIG. 1-3 taken along line 6C-6C of FIG. 3;

FIG. 7 is another perspective view of the test strip of FIGS. 1 and 2;

FIG. 8 a simplified top view of the test strip of FIGs. 1-2, focused on the test strip's first edge indentation and the second edge indentation;

FIG. 9 is a simplified top view depiction of the test strip of FIGS. 1-7 being inserted into a test strip holder of an associated meter, with dashed lines indicating portions of the test strip that are hidden from view;

FIG. 10A is a simplified end view depiction of the test strip of FIGS. 1-7 being inserted into a test strip holder of an associated meter, with dashed lines indicating a sample application area;

FIG. 10B is a simplified cross-sectional end view depiction of the test strip of FIGS. 1-7, being inserted into a test strip holder of an associated meter, taken along line 10B-10B of FIG. 9);

FIGs. 11A and 11B are exploded and perspective views, respectively, of a test strip according to another exemplary embodiment of the present invention;

FIGs. 12A and 12B are exploded and perspective views, respectively, of a test strip according to yet another exemplary embodiment of the present invention;

FIGs. 13A and 13B are exploded and perspective views of a test strip according to yet another exemplary embodiment of the present invention that includes an electrochemical cell;

FIGs. 14A and 14B are simplified bottom and end view depictions, respectively, of a test strip according to one exemplary embodiment of the present invention that includes a sharp-edged border;

FIGs. 15A and 15B are simplified exploded and perspective views, respectively, of a test strip according to a further exemplary embodiment of the present invention;

FIG. 16 is a partial top view of the test strip of FIGs. 15A and 15B (with dashed lines indicating elements that are hidden from view); and

FIG. 17 is a partial cross-sectional view of the test strips of FIGs. 15A and 15B taken along line 17A-17A of FIG. 16.

### DETAILED DESCRIPTION OF THE INVENTION

To be consistent throughout the present specification and for clear understanding of the present invention, the following definitions are hereby provided for terms used therein:

The term "hydrophilic" refers to a surface property of a material that results in a contact angle of less than 90 degrees for an aqueous liquid sample of interest.

The term "hydrophobic" refers to a surface property of a material that results in a contact angle of 90 degrees or greater for an aqueous liquid sample of interest.

The term "highly wettable" refers to a surface property of a material that results in a contact angle of less than approximately 30 degrees for an aqueous liquid sample of interest.

The term "capillary channel" refers to a channel that, due to its geometry and surface properties, provides for movement of a liquid sample therethrough via capillary action.

The term "sharp-edged" refers to an edge with an internal angle no greater than approximately 110 degrees. Under this definition, an edge becomes less sharp as the internal angle increases and the range of internal angles for a sharp-edged feature ranges from greater than 0 degrees up to approximately 110 degrees.

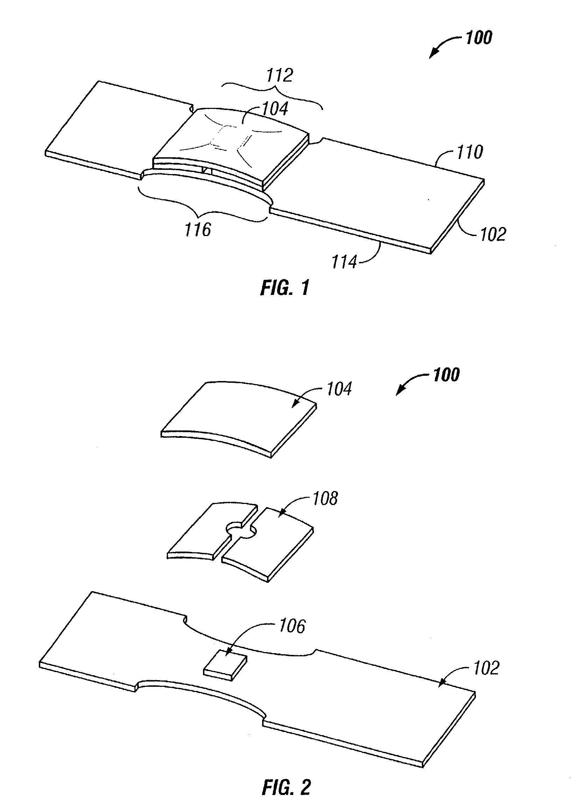

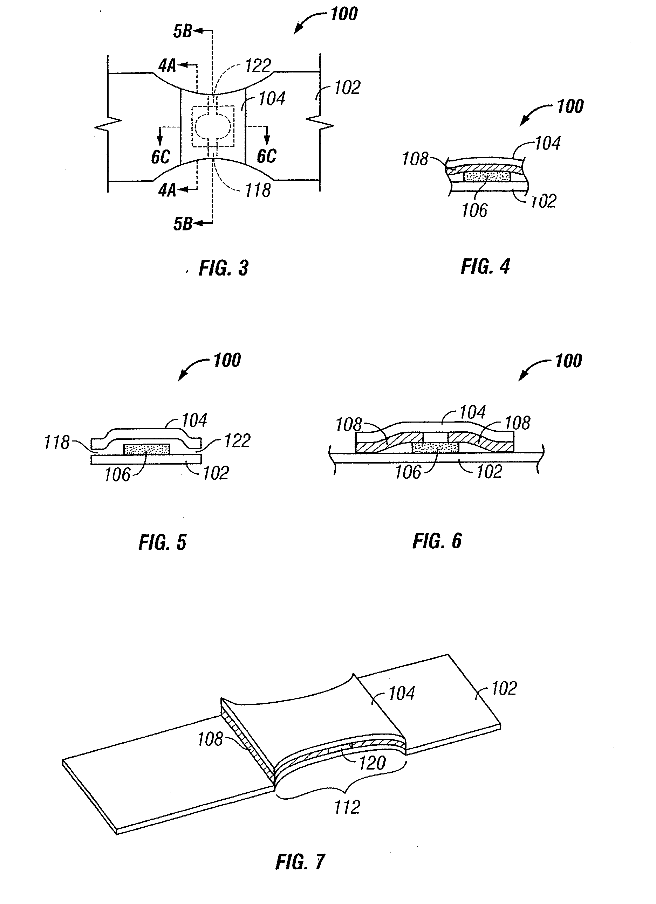

FIGs. 1 and 2 are a perspective and exploded view, respectively, of a test strip 100 (i.e., an ambidextrous capillary-filled test strip) for the detection of an analyte (e.g., blood glucose) in a liquid sample (e.g., whole blood) according to the present invention. FIGs. 3-7 are further illustrations of test strip 100. Test strip 100 includes a base portion 102, an upper portion 104,0 a reagent pad 106 (e.g., a colorimetric reagent pad) and a spacer layer 108, as shown in FIGs. 1-7. In the embodiment of FIG. 1, reagent pad 106 and spacer layer 108 are disposed between the base portion 102 and the upper portion 104.

Test strip 100 also has a first edge 110 with a first edge indentation 112 and a second edge 114 with a second edge indentation 116. First and second edges 110 and 114 are in an opposing relationship (i.e., from the perspective of FIG. 1, first and second edges 110 and 114 are on opposing right-hand and left-hand longitudinal edges of elongated test strip 100, respectively).

Test strip 100 also includes a first capillary channel 118 disposed between base portion 102 and the upper portion 104, as illustrated in FIG. 3. First capillary channel 118 is adapted for providing fluid communication between a first capillary channel inlet 120 (shown in FIG. 7) at first edge indentation 112 and the reagent pad 106. Test strip 100 also has a second capillary channel 122 disposed between the base portion 102 and the upper portion 104. Second capillary channel 122 is adapted for providing fluid communication between a second capillary channel inlet (not shown) at the second edge indentation 116 and reagent pad 106.

Base portion 102 and upper portion 104 can be formed of any suitable material known to one skilled in the art. For example, base portion 102 can be formed of a relatively rigid, non-porous plastic material. It can be desirable to form base portion 102 from a hydrophobic material to help minimize the potential of a liquid sample wetting the lower surface of base portion 102. However, in embodiments where base portion 102 serves to define the first and second capillary channels (as in FIGs. 1-7), it can be beneficial that the upper surface of base portion 102 that defines the first and second capillary channels be either highly wettable or hydrophilic. Furthermore, if desired, base portion 102 can be formed of a clear material (e.g., a clear plastic) to enable detection of color changes at reagent pad 106. Suitable materials for base portion 102 include, but are not limited to, polyester (e.g., Melinex and Mylar), polystyrene, polycarbonate, acrylic and cellulose ester plastic materials. A typical thickness for base portion 102 is, for example, in the range of 0.005 inches to 0.020 inches.

Base portion 102 can optionally include an opening (not shown in FIGs. 1-7) underneath of reagent pad 106 to allow oxygen to reach reagent pad 106. Such an opening can be useful in the circumstance that reagent pad 106 has oxygen requiring reagent(s) incorporated therein.

Upper portion 104 can be formed, for example, from the same material as base portion 102. However, since, in the embodiment of FIGS. 1-7, upper portion 104 also serves to define the first and second capillary channels, it can be beneficial for the lower surface of upper portion 102 that defines the first and second capillary channels to be highly wettable or hydrophilic. One skilled in the art will recognize that a surface of a hydrophobic material can be made hydrophilic and/or wettable by, for example, coating the surface with a suitable hydrophilic or highly wettable material. A typical thickness for upper portion 104 is, for example, in the range of 0.001 inches to 0.020 inches.

Reagent pad 106 can be any suitable reagent pad known in the art, such as a colorimetric reagent pad (e.g., a colorimetric reagent-impregnated membrane) for the detection of blood glucose in a whole blood sample. Suitable reagents for incorporation in a reagent pad include, for example, colorimetric reagents based on (i) a glucose oxidase (GO), horseradish peroxidase (HRPO), 3-methyl-2-benzothiazolinone hydrazone (MBTH) and dimethylamino benzoic acid (DMAB) chemistry; (ii) a GO, phenazine methosulfate (PMS), and 2,2'-Dibenzothiazolyl-5,5'-bis[4-di(2-sulfoethyl)carbamoylphenyl]-3,3'-(3,3'-dimethoxy-4,4'-biphyenylene)ditetrazolium disodium salt (available as WST-5 from Dojindo Molecular Technologies, Gathersburg, MD) chemistry and (iii) a GDH, PMS and WST-5 chemistry. Although reagent pad 106 is illustrated as a single layer, it is envisioned that reagent pad 106 can be either a single-layered or a multi-layered reagent pad. Descriptions of suitable reagents and reagent pads are present in U.S. Patent Nos. 4,900,666, 5,304,468, and 6,420,128, each of which is hereby fully incorporated by reference.

Suitable membranes for use as reagent pads include, for example, U.S. Filter BTS-30 membrane material (an asymmetrical polysolfone membrane), Pall Biodyne membrane material (a symmetrical Nylon membrane with a polyester inner support) and a Millipore Hi-Flow Plus membrane material (a membrane formed from nitrocellulose cast on a Mylar film). A typical thickness for the reagent pad is, for example, in the range of 0.003 inches to 0.010 inches.

Spacer layer 108 can be formed of any suitable material known to one skilled in the art. Furthermore, spacer layer 108 can be an adhesive layer (e.g., a double-sided adhesive tape or an adhesive transfer tape) that serves to affix the upper portion, base portion and reagent pad together during the manufacturing of test strip 100. Suitable materials for spacer layer 108 include, for example, 3M 415 adhesive material (an acrylic/polyester double-sided adhesive tape), 3M 465 adhesive material (an acrylic adhesive transfer tape) and Flexcryl GP3 (an acrylic adhesive emulsion). A typical thickness for the adhesive layer is, for example, in the range of 0.0001 inches to 0.01 inches. For the circumstance where spacer layer 108 is formed from a double-sided adhesive layer, a particularly useful thickness is in the range of 0.002 inches to 0.005 inches.

In the embodiment of FIGS. 1-7, a first capillary channel 118 and a second capillary channel 122 are defined by a lower surface of upper portion 104, an upper surface of base portion 102 and by spacer layer 108. As shown in FIGs. 2 and 3, the first and second capillary channels meet at an enlarged circular capillary area that serves to provide for an even distribution of a liquid sample over reagent pad 106. One skilled in the art will recognize, however, that such an enlarged circular area is an optional feature of test strips according to embodiments of the present invention.

During use of test strip 100, a liquid sample (e.g., a whole blood sample expressed from a fingertip or alternate target site) is applied to one of the first capillary channel inlet 122 or second capillary channel inlet. The liquid sample is then transported, via capillary action, through the capillary channel associated with the inlet where the liquid sample was applied to the reagent pad. As the liquid sample is being transported, the opposing capillary channel can, if need be, function as a vent. The transportation of the liquid sample from the capillary channel inlet to the reagent pad (or other analytical portion in alternative embodiments of the present invention) via either the first or second capillary channel is referred to as "capillary filling" of the test strip.

One skilled in the art will recognize that the first and second capillary channels of test strips according to exemplary embodiments of the present invention can be configured of various dimensions and defined in a variety of manners while still maintaining the ability to function as a capillary. That is that such first and second capillary channels can be defined and of a suitable dimension to provide for the movement of a liquid sample from the capillary channel's inlet to a reagent pad (or other analytical portion) via capillary action. Exemplary dimensions of the capillary channels are a width in the range of 0.005-0.1 inches and a height in the range of less than approximately 0.010 inches. Furthermore, to provide for the use of a liquid sample of relatively small volume, the length of the capillary channels can be, for example, in the range of 0.005 inches to 0.4 inches.

To enable movement of the liquid sample via capillary action, at least one the surfaces defining each of the first and second capillary channels should be hydrophilic in nature. The remaining surface(s) defining the first and second capillary channels can be either hydrophilic or hydrophobic, so long as the capillary channels provide for the movement of a liquid sample therethrough via capillary action. In the embodiment of FIGS. 1-7, the surfaces of the base portion and upper portion that serve to define the first and second capillary channels can both be equally hydrophilic nature, one of the surfaces can be more hydrophilic than the other surface, or one of the surfaces can be hydrophilic (for example, highly wettable) while the other is hydrophobic.

Test strips according to exemplary embodiments of the present invention, including test strip 100, provide several benefits. First, since a liquid sample can be applied to the inlet of either the first or the second capillary channels, test strip 100 can be easily manipulated by both left-handed and right-handed users (accordingly, the present test strips can be referred to as "ambidextrous" test strips). For example, a user can apply a liquid sample to test strip 100 by gripping base portion 102 in one hand and then bringing test strip 100 into contact with a target site, from which a liquid sample has been expressed (e.g., a fingertip target site of the opposite hand, from which a whole blood sample has been expressed). The test strip can then be inserted into an associated meter for detection of an analyte (e.g., blood glucose) in the liquid sample. Such a procedure is referred to as "off-meter dosing" since the liquid sample is applied to the test strip prior to the test strip being inserted into a meter.

Second, since the first and second capillary channels need only extend from the reagent pad to the first or second edge indentation, the length of the first and second capillary channels can be minimized, thereby enabling the use of a liquid sample with a relatively small volume (e.g., a volume in the range of 0.1 micro-liters to 15 micro-liters).

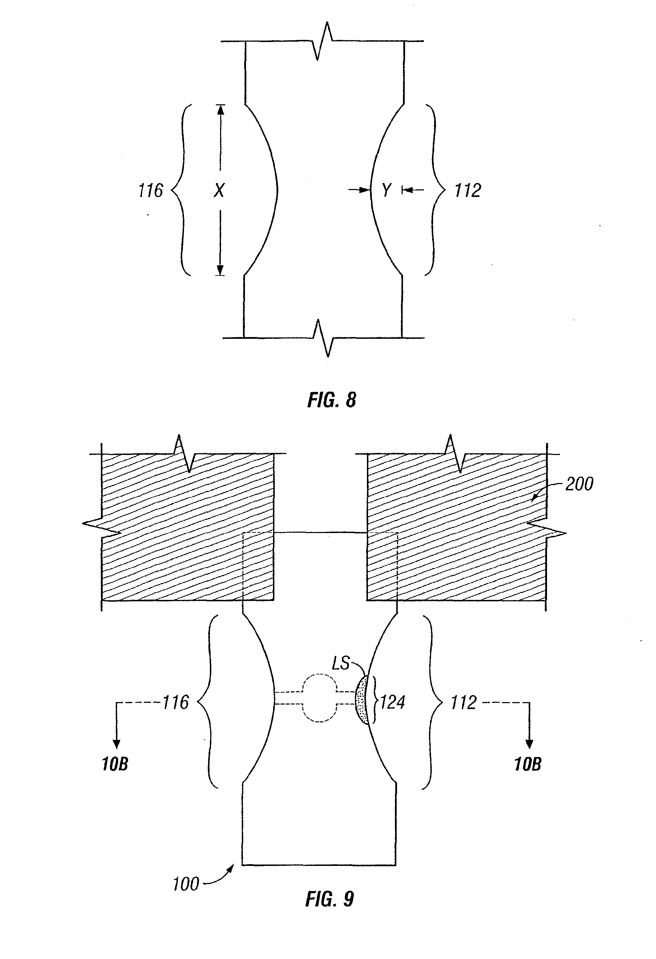

Third, the profile of the first and second edge indentations can be configured to accommodate a variety of target sites (e.g., a finger or forearm target site) and, thereby, guide a user in accurately applying a liquid sample to one of the first and second capillary channels. FIG. 8 is a simplified top view of a portion of test strip 100 focusing on first edge indentation 112 and second edge indentation 116. In this embodiment, first and second edge indentations 112, 116 are curved with a "length" X in the range of 0.15 inches to 1.0 inches (e.g., 0.50 inches) and a "depth" Y in the range of 0.05 inches to 0.20 inches (e.g., 0.12 inches). This particular geometric configuration of the first and second edge indentation can be employed to efficiently guide a fingertip target site to the first and second capillary channels, respectively. Once apprised of the present disclosure, one skilled in the art will recognize that other indentations with other geometric configurations (e.g., rectangular shaped indentations) and sizes can also be beneficially employed to suit alternate target sites, such as forearms and earlobes.

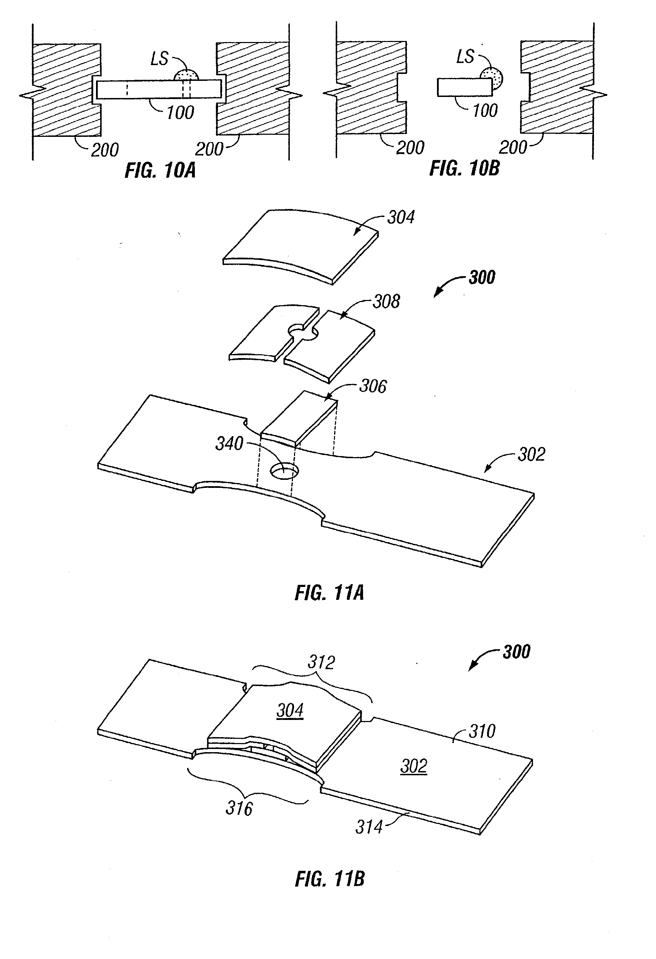

Fourth, since a liquid sample is applied within either the first edge indentation or second edge indentation, test strips according to exemplary embodiments of the present invention can be gripped by an associated meter in such a way that the liquid sample (e.g., a whole blood sample) cannot contaminate the meter. FIGs. 9 and 10 are simplified depictions of test strip 100 being inserted into a test strip holder 200 of an associated meter (not shown).

FIG. 9 depicts test strip 100 following application of a liquid sample to first capillary channel inlet 120 within first edge indentation 112. A region surrounding first capillary channel inlet 120 and within first edge indentation 112 (referred to a "sample application region" 124) is shown as being contaminated with liquid sample. Although not illustrated in FIG. 9, one skilled in the art will appreciate that a similar sample application region exists surrounding the second capillary channel inlet within the second edge indentation 116. Such a sample application area would typically have a dimension along the edge of the test strip in the range of 0.010 inches to 0.20 inches. In addition, excess liquid sample could extend outwardly from the sample application region for a distance of, for example, 0.05 inches.

As illustrated in FIGs. 9, 10A and 10B, test strip holder 200 is configured to receive test strip 100 in the absence of contact between the sample application region 124 of the first edge indentation 112 and the test strip holder 200. Furthermore, test strip holder 200 is configured to receive test strip 100 in the absence of contact between the sample application region of the second edge indentation and the test strip holder. The absence of such contact prevents any liquid sample (LS in FIGs. 9, 10A and 10B) that may have inadvertently contaminated the sample application region from subsequently contaminating the test strip holder or meter.

FIGs. 11A and 11B are exploded and perspective views, respectively, of a test strip 300 for the detection of an analyte (e.g., blood glucose) in a liquid sample (e.g., a whole blood sample) according to the present invention. Test strip 300 includes a base portion 302, an upper portion 304, a reagent pad 306 (e.g., a reagent-impregnated membrane) and a spacer layer 308, as illustrated in FIGs. 11A and 11B. In the embodiment of FIGs. 11A and 11B, reagent pad 306 is sized such that it extends across the entire width of test strip 300. Test strip 300 also has a first edge 310 with a first edge indentation 312 and a second edge 314 with a second edge indentation 316. First edge 310 and second edge 314 are in an opposing relationship (i.e., from the perspective of FIGs. 11A and 11B, first and second edges 310 and 314 are on opposing right-hand and left-hand longitudinal edges of elongated test strip 300, respectively). Base portion 302 of test strip 300 includes an opening 340 for the provision of allowing oxygen to reach reagent pad 306. Optionally, if there is no need to provide oxygen to reagent pad 306, base portion 302 can be formed of a clear material to allow detection of a color change at reagent pad 306.

In the embodiment of FIGs. 11A and 11B, a first capillary channel and a second capillary channel that provide fluid communication between the first edge indentation 312 and the reagent pad 306 and between the second edge indentation 316 and the reagent pad 306, respectively, are defined by upper portion 304, spacer layer 308 and reagent pad 306. If desired, an adhesive layer (not shown) can be positioned between the reagent pad and the base portion to prevent the formation of a variable fluid film between the reagent pad and the base portion.

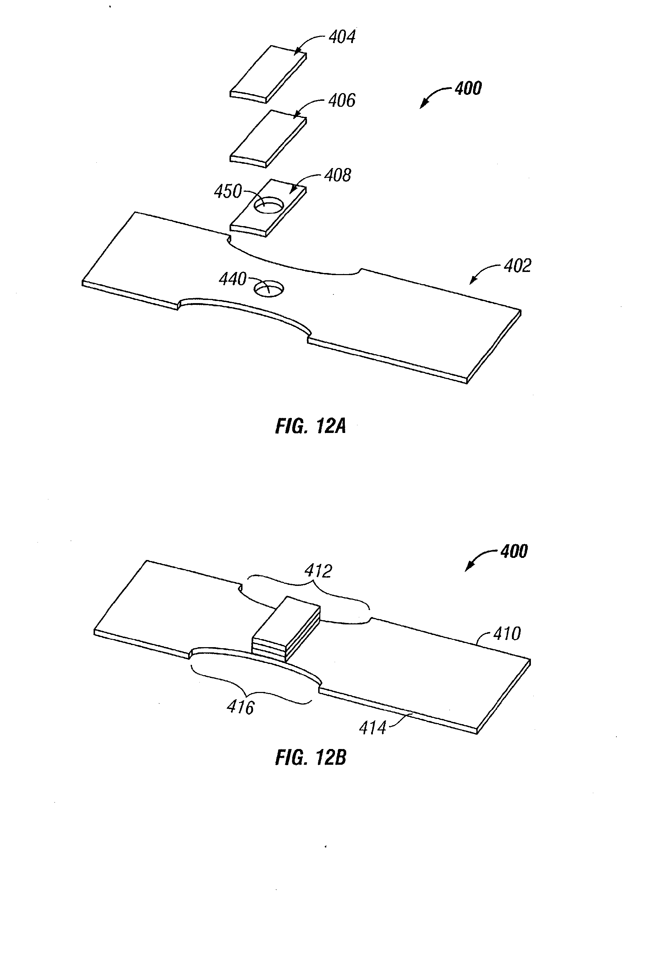

FIGs. 12A and 12B are exploded and perspective views, respectively, of a test strip 400 for the detection of an analyte (such as blood glucose) in a liquid sample (e.g., a whole blood sample) according to the present invention. Test strip 400 includes a base portion 402, an upper portion 404, a reagent pad 406 (e.g., a reagent-impregnated membrane), and a double-sided adhesive layer 408, as illustrated in FIGs. 12A and 12B.

Test strip 400 also has a first edge 410 with a first edge indentation 412 and a second edge 414 with a second edge indentation 416. First edge 410 and second edge 414 are in an opposing relationship (i.e., from the perspective of FIGs. 12A and 12B, first and second edges 410 and 414 are on opposing right-hand and left-hand longitudinal edges of elongated test strip 400, respectively). Base portion 402 and double-sided adhesive layer 408 each include an opening (openings 440 and 450, respectively) for the provision of allowing oxygen to reach reagent pad 406.

In the embodiment of FIGs. 12A and 12B, a first capillary channel and a second capillary channel that enable fluid communication between the first edge indentation 412 and the reagent pad 406 and between the second edge indentation 414 and the reagent pad 406, respectively, are provided by reagent pad 406 itself. This is accomplished by forming reagent pad 406 from a material that is adapted for lateral capillary flow of a liquid sample from either the first or second edge indentation to throughout the reagent pad. Any suitable material that provides such a lateral capillary flow can be employed for reagent pad 406, including, for example, Millipore Hi-Flow membrane material. Upper portion 404 can be formed from, for example, a single-sided adhesive material.

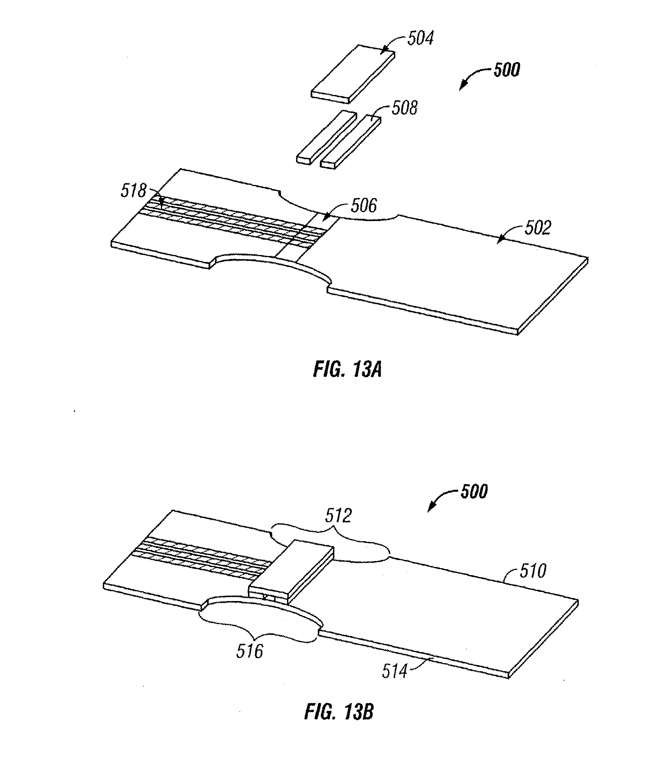

FIGs. 13A and 13B are exploded and perspective views, respectively, of a test strip 500 for the electrochemical detection of an analyte (such as blood glucose) in a liquid sample (e.g., a whole blood sample) according to an exemplary embodiment of the present invention. Test strip 500 includes a base portion 502, an upper portion 504, an electrochemical reagent application zone 506, electrode traces 518 and a spacer layer 508, as illustrated in FIGs. 13A and 13B. Test strip 500 also has a first edge 510 with a first edge indentation 512 and a second edge 514 with a second edge indentation 516. First edge 510 and second edge 514 are in an opposing relationship (i.e., from the perspective of FIGs. 13A and 13B, first and second edges 510 and 514 are on opposing right-hand and left-hand longitudinal edges of elongated test strip 500, respectively).

In the embodiment of FIGs. 13A and 13B, an electrochemical cell is defined by spacer layer 508 and electrode traces 518. In addition, a first capillary channel and a second capillary channel that provide fluid communication between the first edge indentation 512 and the electrochemical cell and between the second edge indentation 516 and the electrochemical cell, respectively, are defined by upper portion 504, spacer layer 508 and base portion 502.

Test strip 500 can include any suitable electrochemical cell reagent known in the art in electrochemical reagent application zone 506. An exemplary electrochemical cell reagent is a mixture of GDH and potassium ferricyanide (K₃Fe(CN)₆).
Furthermore, any electrochemical cell configuration known to those skilled in the art as suitable for the detection of an analyte in a liquid sample can be employed in test strip 500.

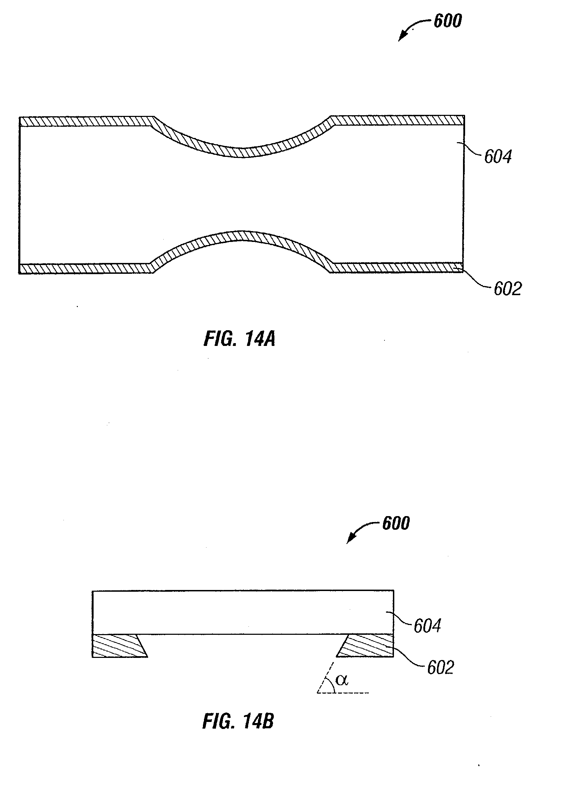

During use of test strips for the detection of an analyte in a liquid sample, it is possible that a liquid sample applied to the test strip may migrate to the bottom surface of a test strip. Such migration can interfere with the measurement of a property or properties of the test strip (e.g., an optical reflectance, optical transmittance, optical absorption or an electrochemical analysis) by an associated meter. FIGs. 14A and 14B are simplified bottom and end views, respectively, of a test strip 600 that includes a sharp-edged border 602 disposed on a bottom surface 604 of the test strip.

Sharp-edged border 602 has an internal angle (angle "α" in FIG. 14B) of between 0 degrees and 110 degrees. Such a sharp-edged border prevents any liquid sample that is inadvertently transferred to the sharp-edged border from easily migrating to the bottom surface 604 of test strip 600. Sharp-edged border 602 can be formed from any suitable material. However, if sharp-edged border 602 is formed of a hydrophobic material or treated to render its surface hydrophobic, additional protection against the migration of a liquid sample across the sharp-edged border to the bottom surface of the test strip is provided.

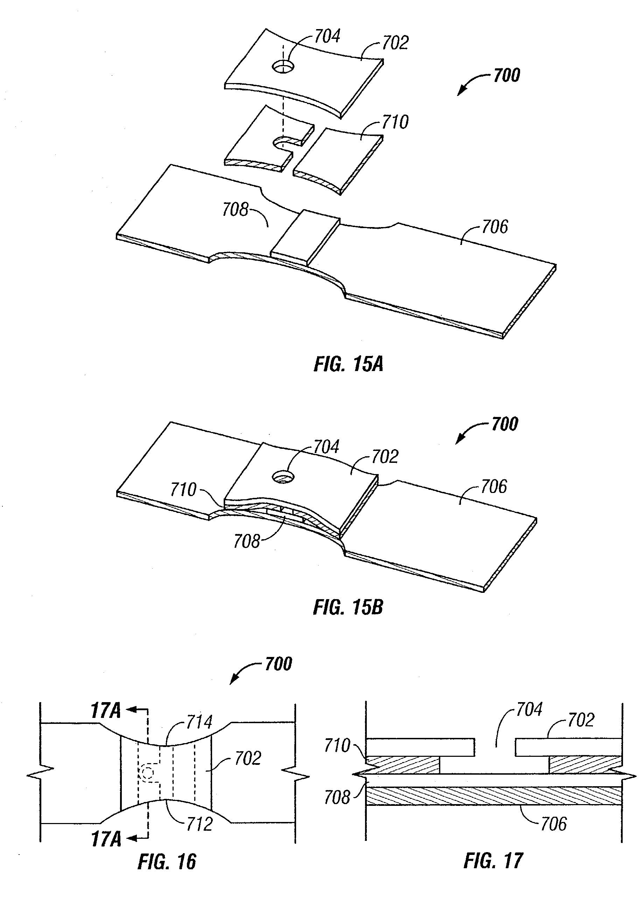

FIGs. 15A, 15B, 16 and 17 depict a test strip 700 for the detection of an analyte in a liquid sample according to a further exemplary embodiment of the present invention. Test strip 700 includes an upper portion 702 with an opening 704, a base portion 706, a reagent pad 708 (e.g., a colorimetric reagent pad) and a spacer layer 710. In the embodiment of FIGS. 15A - 17, reagent pad 708 and spacer layer 710 are disposed between base portion 706 and upper portion 702. Test strip 700 also includes first capillary channel 712 and second capillary channel 714, both of which are adapted for providing fluid (i.e., gas or liquid) communication between reagent pad 708 and an edge of test strip 700 (as described previously with respect to the embodiment of FIGS. 1-7).

A liquid sample (e.g., a whole blood sample) can be applied (i.e., "dosed") either from the top of test strip 700 through opening 704 or from either the left or right hand edge of the test strip via first capillary channel 712 or second capillary channel 714, respectively. When a liquid sample is applied from the top of test strip 700 via opening 704, the first and second capillary channels act as vents. When a liquid sample is applied using either the first or second capillary channel, opening 704 can serve as a vent along with the capillary channel to which the liquid sample was not applied. Since a liquid sample can be applied to test strip 700 in any of the three above described manners, users of limited dexterity can easily employ test strip 700.

In the embodiments of FIGs. 15A-17, opening 704 is positioned in upper portion 702 such that opening 704 is off-center with respect to reagent pad 708. By placing opening 704 off-center, a liquid sample applied via opening 704 can wet a read area (not shown) located in the middle of reagent pad 708 without opening 704 obstructing the read area. A typical diameter for opening 704 is in the range of 0.02 to 0.125 inches in diameter.

Upper portion 702 can, if desired, be coated with a material that modifies its hydrophilicity. Such a coating can optimize liquid sample flow through opening 704 toward reagent pad 708. For example, the upper surface of upper portion 702 can be coated with a hydrophobic coating to make the interior of opening 704 more attractive for a liquid sample than the upper surface of upper portion 702 itself, thereby optimizing liquid sample flow through opening 704.

Spacer layer 710 can be formed of, for example, a pressure sensitive adhesive using techniques known to those skilled in the art, including screen-printing techniques, gravure coating techniques, slot coating techniques, die cutting, laser scribing or punching an adhesive material before lamination onto the underside of the upper portion. The adhesive of such a spacer layer can be, for example, a double-sided pressure sensitive adhesive, a UV cured adhesive, a heat activated adhesive or a thermosetting plastic. As a non-limiting example, spacer layer 710 can be formed by screen printing a pressure sensitive adhesive, such as part number A6435 water-based acrylic copolymer pressure sensitive adhesive commercially available from Tape Specialties LTD, Tring, Herts, United Kingdom.

Employing a printed adhesive layer as a spacer layer is beneficial since the thickness of the spacer layer can be minimized. For example, a typical printed adhesive layer can have a thickness of less than 0.005 inches. A thinner adhesive layer is desirable because this minimizes the distance a liquid sample must travel from opening 704 in upper portion 702 to reagent pad 708.

It should be understood that various alternatives to the embodiments of the invention described herein may be employed in practicing the invention. It is intended that the following claims define the scope of the invention and that structures within the scope of these claims and their equivalents be covered thereby.

## Claims

1. A test strip for detecting an analyte in a liquid sample, the test strip comprising:
an analytical portion;
a first edge with a first edge indentation;
a second edge with a second edge indentation;
a first capillary channel adapted for providing fluid communication between the first edge indentation and the analytical portion; and
a second capillary channel adapted for providing fluid communication between the second edge indentation and the analytical portion,
wherein the first edge and second edge are in an opposing relationship.

2. The test strip of claim 1, wherein:
the first edge indentation includes a sample application region;
the second edge indentation includes a sample application region;
the first capillary channel is adapted to provide fluid communication between the sample application region of the first edge indentation and the analytical portion; and
the second capillary channel is adapted to provide fluid communication between the sample application region of the second edge indentation and the analytical portion.

3. The test strip of claim 1 or claim 2, wherein the test strip further includes a sharp-edged border disposed on a bottom surface of the test strip.

4. The test strip of claim 3, wherein the sharp-edged border is adapted to prevent the migration of the liquid sample to the bottom surface of the test strip.

5. The test strip any one of claims 1 to 4 further including:
a base portion; and
an upper portion;
wherein the analytical portion, first capillary channel and second capillary channel are disposed between the base portion and the upper portion.

6. The test strip of claim 5, wherein the upper portion includes an opening for applying a liquid sample to the test strip.

7. The test strip of claim 5 or claim 6, further including a spacer layer and wherein the spacer layer, base portion and upper portion serve to define the first and second capillary channels.

8. The test strip of any one of claims 1 to 7, wherein the first edge indentation and the second edge indentation are curved edge indentations configured to accommodate a target site.

9. The test strip of claim 8, wherein the first edge indentation and the second edge indentation are each configured to accommodate a fingertip target site.

10. The test strip of claim 8, wherein the first edge indentation and second edge indentation are each configured to accommodate a forearm target site.

11. The test strip of any one of claims 1 to 10, wherein the first and the second edge indentations are rectangular in shape.

12. The test strip of any one of claims 1 to 11, wherein the analytical portion includes a reagent pad.

13. The test strip of claim 12, wherein said base portion contains an opening underneath said reagent pad.

14. The test strip of any one of claims 1 to 11, wherein the analytical portion includes an electrochemical cell.

15. The test strip of any one of claims 1 to 14, wherein the first and second capillary channels are adapted to accommodate a liquid sample with a volume in the range of 0.1 micro-liters to 15 micro-liters.

16. The test strip of any one of claims 1 to 15, wherein the test strip is an elongated test strip and the first edge and second edge are opposing longitudinal edges.

17. The test strip of any one of claims 1 to 16, wherein the liquid sample is a whole blood sample.

18. The test strip of claim 17, wherein the analyte is blood glucose.

19. A test strip and meter combination for detecting an analyte in a liquid sample, the test strip and meter combination comprising:
a test strip according to any one of claims 1 to 18; and
a meter that includes a test strip holder, the test strip holder being configured to receive the test strip in the absence of contact between the sample application region of the first edge indentation and the test strip holder and between the sample application region of the second edge indentation and the test strip holder.
